# EUROPÄISCHE PATENTSCHRIFT

(11) **EP 0 754 944 B1**
(45) Veröffentlichungstag und Bekanntmachung des Hinweises auf die Patenterteilung: **12.01.2000**
(21) Anmeldenummer: 96890126.4
(22) Anmeldetag: 17.07.1996
(51) Int. Cl.: G01N 33/483

(54) **Verfahren zur Qualitätskontrolle eines portablen Analysensystems**
Method for the quality control of a portable analyser
Procédé pour le contrôle de qualité d'un analyseur portable

(30) Priorität: 17.07.1995 AT 121595
(43) Veröffentlichungstag der Anmeldung: 22.01.1997
(73) Patentinhaber: AVL Medical Instruments AG, 8207 Schaffhausen (CH)
(72) Erfinder: Leiner, Marco Jean-Pierre, Dr., 8045 Graz (AT); Tusa, James K., Alpharetta, A 30201 (US)
(74) Vertreter: Babeluk, Michael, Dipl.-Ing. Mag., Patentanwälte Babeluk - Krause

(56) Entgegenhaltungen:
- EP-A- 0 395 384
- WO-A-86/05590
- US-A- 4 135 883

## Beschreibung

Die vorliegende Erfindung betrifft ein Verfahren zur Qualitätskontrolle eines Analysensystems bestehend aus einem portablen Analysator mit einsetzbaren Einmal-Kassetten, deren Probenraum optische und/oder elektrochemische Sensoren aufweist, wobei die Sensoren entweder trocken oder in Kontakt mit einem flüssigen Konditionsmedium gelagert werden. Es handelt sich dabei insbesondere um Sensoren, die zur Bestimmung klinisch relevanter Parameter (pH, pC02, p02, Ionen, (z. B. Li+, Na+, K+, Mg++, Ca++, Cl-) und Enzymsubstraten ( z. B. Glucose, Lactat, Harnstoff) in biologischen Flüssigkeiten vorgesehen sind.

Zum besseren Verständnis der Erfindung werden nachstehend einige wichtige Begriffe definiert:

Analysator: Meßinstrument basierend auf optischen und/oder elektrochemischen Sensoren, zur Vermessung von Proben (z.B. Blutproben)

Portabler Analysator: Point-of-Care-Meßinstrument basierend auf optischen und/oder elektrochemischen Sensoren. Die Sensoren sind in Einmal-Kassetten eingebaut, welche zur Probenmessung in den Analysator eingesetzt und nach der Messung entsorgt werden.

Sensor: Optisches oder elektrochemisches Meßelement zur Bestimmung der Konzentration bzw. des Partialdruckes zumindest einer, in einer Flüssigkeit gelösten chemischen Komponente. Mehrere Sensoren können zur simultanen Bestimmung der Konzentrationen bzw. der Partialdrücke verschiedener chemischer Komponenten verwendet werden.

Simultane Messung: Die Sensoren einer Sensoranordnung werden in ein und demselben Verfahrensschritt mit dem zu messenden Medium (Kalibriermedium, Kontrollflüssigkeit, Probe) in Kontakt gebracht. Die Erfassung der Meßsignale muß nicht unbedingt zeitgleich erfolgen.

Einmal-Kassette: Mit einem portablen Analysator mechanisch sowie elektrisch und/oder optisch in Kontakt zu bringendes Teil, welches zumindest einen Probenraum und mit diesem in zumindest indirekten Kontakt stehende Sensoren aufweist. Die Einmal-Kassette ist zur Messung einer einzigen Probe ausgelegt.

Konditionierung: Zeitlicher Kontakt der Sensoren mit einem gegebenen Medium zu Erlangung einer stabilen Sensorkennlinie. Trocken gelagerte Sensoren weisen nach dem Kontakt mit wäßrigen oder feuchten gasförmigen Medien eine sich zeitlich ändernde Kennlinie auf. Einen ähnlichen Effekt bewirkt, insbesondere bei Sensoren mit ionenpermeablen Materialien, der diffusionsbedingte Austausch chemischer Komponenten mit verschieden zusammengesetzten wäßrigen Medien.

Sensorkennlinie: Funktionaler Zusammenhang zwischen der Konzentration (bzw. dem Partialdruck) einer, in einer wäßrigen Flüssigkeit vorliegenden chemischen Komponente (unabhängige Variable) und der Größe eines meßbaren optischen bzw. elektrischen Signals eines optischen bzw. elektrochemischen Sensors.

Kalibration: Ermittlung der Sensorkennlinie.

1-Punkt Kalibration: Kalibration bei einem Erwartungswert der Meßgröße. Am häufigsten ist zur Kalibration die Signalermittlung bei zumindest zwei Werten der Meßgröße erforderlich. Ist hingegen der funktionale Verlauf der Kennlinie zumindest teilweise bekannt (Batchweise Erhebung bei der Fertigung oder reproduzierbare Fertigung), so ist zur vollständigen Kalibration eine Signalermittlung bei einem Wert der Meßgröße zumeist ausreichend.

Kalibriermedium, Kalibrierflüssigkeit: Wäßrige Flüssigkeit oder Gasgemisch, welches zumindest eine, mit einer Sensoranordnung zu bestimmende chemische Komponente mit bekannter Konzentration bzw. mit bekanntem Gaspartialdruck enthält, welche(r) einem Erwartungswert der entsprechenden chemischen Komponente(n) in der jeweiligen Meßsituation entspricht.

Qualitätskontrolle: Simultane Bestimmung der Konzentrationen gelöster chemischer Substanzen und/oder Gaspartialdrücke einer Qualitätskontrollflüssigkeit mit einer Sensoranordnung (z.B. Einmal-Kassette) mit anschließendem Vergleich der Ist- und Sollwerte:

Qualitätskontrollflüssigkeit: In einem, vorzugsweise für Gase (z.B., O2, CO2, N2), Wasser, und in wäßrigen Systemen lösliche chemische Substanzen, impermeablen Gebinde (vorzugsweise Glas) vorliegende, wäßrige Flüssigkeit, welche mit einem Gas bekannter Zusammensetzung beaufschlagt ist, und verschiedene, mit einer Sensoranordnung zu bestimmende chemische Komponenten eines flüssigen Meßgutes in einer Konzentration enthält, welche einem Erwartungswert der Meßgröße in der jeweiligen Meßsituation entspricht.

Meßgut: Flüssigkeiten (beispielsweise biologische Flüssigkeiten, wie Vollblut, Serum, Harn). Konzentrationswerte bzw. Gaspartialdrücke der zu bestimmenden Komponenten essentiell unbekannt.

Probe: Stichprobe des Meßgutes.

Probenmessung: Simultane Bestimmung der Konzentrationen und/oder Gaspartialdrücke gelöster chemischer Substanzen einer Probe mit einer Sensoranordnung (z. B. Einmal-Kassette).

In klinischen Labors werden verschiedene Analysatoren zur in-vitro Analyse biologischer Flüssigkeiten (Blut, Harn, Plasma, Serum) verwendet. Die Instrumente beruhen zumeist auf elektrochemischen Sensoren welche, vor der eigentlichen Messung, mittels im Instrument vorliegender flüssiger und gasförmiger Kalibriermedien, zumindest bei einem Erwartungswert der Meßgrößen kalibriert werden. Zur Kalibrierung der einzelnen Sensoren werden verschiedene flüssige und gasförmige Kalibriermedien eingesetzt.

Vielfach ist es erforderlich, Genauigkeit und Zuverlässigkeit dieser Meßsysteme mittels Qualitätskontrollflüssigkeiten zur überprüfen. Diese sind, zumeist in Glasgebinden dicht verschlossene, und mit einem Gas bekannter Zusammensetzung an 02 und C02 beaufschlagte, wäßrige salzhaltige pH-gepufferte Lösungen. Die Flüssigkeiten können zudem eine Reihe von Additiven zur Erhöhung der Gaslöslichkeit, Tensiden zur Erhöhung der Benetzbarkeit, und keimtötende Chemikalien zur Unterbindung biologischer Aktivitäten enthalten. Qualitätskontrollflüssigkeiten werden vorzugsweise zur simultanen Kontrolle aller, im Instrument vorhandener Sensoren eingesetzt und weichen in ihrer chemischen Zusammensetzung von den verwendeten Kalibriermedien ab.

Im Gegensatz zu den in klinischen Zentrallabors verwendeten Analysatoren (Standgeräte) sind eine Reihe von portablen Analysatoren (Point-of-Care Instrument) bekannt geworden (EP 0 460 343 B1, US-A 5,080,865, WO 92/01928, US-A 5,288,646).

Sie beruhen auf optischen oder elektrochemischen Sensoren, welche in Einmal-Kassetten (disposable cartridge, single-use cartridge) eingebaut sind. Da für jede Analyse eine neue Kassette verwendet wird, unterscheiden sich die Anforderungen an die Sensoren von jenen der Standgeräte. Durch Verwendung nur eines einzigen Kalibriermediums müssen die Sensorkennlinien zumindest teilweise beim Herstellprozeß erhoben werden bzw. durch diesen bekannt sein. Die Kennliniendaten werden jeweils vor der Messung mit einer Kassette in den Analysator eingespeist bzw. liegen im Analysator vor. Durch die kostengüstige Verwendung eines einzigen Kalibriermediums, erfolgt die Kalibration bei einem einzigen Erwartungswert der jeweiligen Meßgröße (1-Punkt Kalibration) und simultan für alle, in der Kassette vorhandenen Sensoren. Die Sensoren sollen sofort, ohne zusätzliche Konditionierungsschritte, nach dem Einlegen einer Einmal-Kassette in den portablen Analysator meßbereit sein.

Einmal-Kassetten können zwar auch mit herkömmlichen Qualitätskontrollflüssigkeiten beaufschlagt werden, wegen des diffusionsbedingten Austausches chemischer Komponenten dieser Kontrollflüssigkeiten mit den Sensoren ist jedoch die anschließende Messung einer Probe mit denselben Sensoren nicht möglich, bzw. erfordert aufwendige zusätzliche Schritte und Maßnahmen.

Da zwischen den zumeist schichtförmig aufgebauten Materialien der Sensoren und den Meßmedien (Kalibriermedium, Kontrollmedium bzw. Probe) ein langsamer diffusionsbedingter Austausch chemischer Substanzen erfolgt, verändert sich die chemische Zusammensetzung der sensitiven Schichten. Infolge veränderter Konzentrationen verändern sich (driften) die Sensorkennlinien. In Abhängigkeit der chemischen Zusammensetzung, Dauer und Temperatur der die Sensoren kontaktierenden Medien erhält man unterschiedliche Meßergebnisse, je nachdem ob die Messung des Meßgutes nach der Kalibration oder nach der Kontrollmessung durchgeführt wird. Somit läßt sich mit bekannten Qualitätskontrollflüssigkeiten bzw. bekannten Verfahren zur Qualitätskontrolle zwar die Zuverlässigkeit und Genauigkeit des Analysators überprüfen, nicht jedoch die Zuverlässigkeit und Genauigkeit einer zur Messung des Meßgutes vorgesehenen Einmal-Kassette und Sensoren.

Aus der EP 0 226 593 B bzw. der WO 86/05590 ist beispielsweise ein Analysensystem zur Vermessung von Blutproben bekanntgeworden, bei welchem eine nach Verwendung wegwerfbare Kartusche vorgesehen ist. Die Kartusche weist einen ersten gasundurchlässigen Behälter für eine Kalibrierflüssigkeit A sowie einen zweiten, ebensolchen Behälter, für eine Kalibrierflüssigkeit B auf, wobei die chemischen Charakteristika beider Kalibrierflüssigkeiten unterschiedlich jedoch bekannt sind. Die beiden Kalibrierflüssigkeiten dienen zur Zweipunktkalibration der Kassette. Eine Qualitätskontrolle im Sinne der eingangs aufgestellten Definition ist nicht vorgesehen.

In der US-A 4,135,883 wird ein Blutanalysator beschrieben, bei welchem in einer Zentrifugiereinrichtung Küvetten mit den Blutproben aufgenommen werden können. Zur Kennzeichnung der Küvetten werden Barcodes verwendet, welche über ein Photodiodenarray eingelesen werden. Weiters ist eine Photodiode zur Bestimmung der Intensität der Transmissionsstrahlung bei Durchstrahlung der Küvette vorgesehen. Der Begriff Sensor wird nur im Zusammenhang mit den Photodioden des Diodenarrays, mit der Definition der Rotorposition sowie mit der Transmissionsmessung verwendet. Im Probenraum d. h. in den Küvetten liegen allerdings keine elektrochemischen bzw. optischen Sensoren vor.

Eine selbstkalibrierende Einmal-Kassette für einen klinischen Analysator ist weiters aus der WO 85/04719 bekanntgeworden. Die Kassette weist ein drehbares Reservoir mit mehreren Kammern auf, von welchen eine eine Kalibrierflüssigkeit und die andere eine Probe beinhaltet. Durch Drehen des Reservoirs werden die einzelnen Kammern zeitlich nacheinander mit dem eigentlichen Meßkanal verbunden, in welchen die gewünschten Parameter für die Kalibrierflüssigkeit und die Probe ermittelt und nachher einer Auswertung zugeführt werden.

Aufgabe der vorliegenden Erfindung ist es, Verfahren zur Qualitätskontrolle anzugeben, welche es bei Verwendung von Einmal-Kassetten ermöglichen, im Anschluß an eine Kontrollmessung, eine Messung mit einem Meßgut mit derselben Einmal-Kassette durchzuführen und zudem Rückschlüsse auf die Zuverlässigkeit des Analysators bzw. auf die Zuverlässigkeit und Genauigkeit der einzelnen, in der Einmal-Kassette vorhandenen Sensoren zu ziehen.

Diese Aufgabe wird für Sensoren, welche in Kontakt mit einem flüssigen Konditioniermedium gelagert werden, erfindungsgemäß dadurch gelöst:
a) daß herstellungsbedingte Kenndaten der einzelnen Sensoren in den Analysator eingelesen werden bzw. gespeichert sind und die Kassette in den Analysator eingesetzt wird,
b) daß die sich im Probenraum befindliche Konditionierflüssigkeit durch ein gasförmiges Kalibriermedium ersetzt wird, wobei in Kontakt mit einzelnen der Sensoren verbleibende Reste der Konditionierflüssigkeit mit dem gasförmigen Kalibriermedium beaufschlagt werden,
c) daß die Kalibrationssignale der Sensoren simultan erfaßt werden,
d) daß das gasförmige Kalibriermedium durch eine Qualitätskontrollflüssigkeit ersetzt wird, welche im Hinblick auf jene chemischen und/oder physikalischen Parameter, welche die Kennlinie zumindest eines Sensors während der Kalibrierung oder Konditionierung beeinflussen, mit der Konditionierflüssigkeit - innerhalb der gewünschten Meßgenauigkeit für die zu messenden Probenbestandteile - übereinstimmt,
e) daß die Kontrollsignale der Sensoren simultan erfaßt werden,
f) daß aus den eingelesenen bzw. gespeicherten Kenndaten und den in den Punkten c) und e) erfaßten Kalibriations- und Kontrollsignalen Ist-Kontrollwerte für die einzelnen Sensoren ermittelt werden,
g) daß die Ist-Kontrollwerte mit den bekannten Soll-Kontrollwerten verglichen werden, und
h) daß anschließend an die Qualitätskontrolle mit derselben Kassette eine Probenmessung durchgeführt wird, falls die Ist-Kontrollwerte mit den Soll-Kontrollwerten innerhalb der gewünschten Meßgenauigkeit übereinstimmen.

In ähnlicher Weise ist erfindungsgemäß für trocken gelagerte Sensoren vorgesehen,
a) daß herstellungsbedingte Kenndaten der einzelnen Sensoren in den Analysator eingelesen werden bzw. gespeichert sind und die Kassette in den Analysator eingesetzt wird,
b) daß die Sensoren mit einem flüssigen Kalibriermedium beaufschlagt werden,
c) daß die Kalibrationssignale der Sensoren simultan erfaßt werden, wobei die Zeitspanne zwischen dem Erstkontakt der Sensoren mit der Kalibrierflüssigkeit und der Erfassung der Kalibrationssignale berücksichtigt wird,
d) daß die Sensoren mit einer Qualitätskontrollflüssigkeit kontaktiert werden, wobei ggf. zur Trennung der Kalibrierflüssigkeit von der Qualitätskontrollflüssigkeit ein Gas bekannter Zusammensetzung eingebracht wird, sowie daß die Qualitätskontrollflüssigkeit im Hinblick auf jene chemischen und/oder physikalischen Parameter, welche die Kennlinie zumindest eines Sensors während der Kalibrierung beeinflussen, mit der Kalibrierflüssigkeit - innerhalb der gewünschten Meßgenauigkeit für die zu messenden Probenbestandteile - übereinstimmt,
e) daß die Kontrollsignale der Sensoren erfaßt werden, wobei die Zeitpanne zwischen dem Erstkontakt der Sensoren mit der Kalibrierflüssigkeit und der Erfassung der Kontrollsignale berücksichtigt wird,
f) daß aus den eingelesenen bzw. gespeicherten Kenndaten und den in den Punkten c) und e) erfaßten Kalibrations- und Kontrollsignalen Ist-Kontrollwerte für die einzelnen Sensoren ermittelt werden,
g) daß die Ist-Kontrollwerte mit den bekannten Soll-Kontrollwerten verglichen werden, und
h) daß anschließend an die Qualitätskontrolle mit derselben Kassette eine Probenmessung durchgeführt wird, falls die Ist-Kontrollwerte mit den Soll-Kontrollwerten innerhalb der gewünschten Meßgenauigkeit übereinstimmen.

In einer vorteilhaften Ausführungsvariante ist vorgesehen, daß die Sensoren mit einer Qualitätskontrollflüssigkeit in Kontakt gebracht werden, welche im interessierenden Meßbereich und bei gegebener Meßtemperatur eine CO2/pH-Gleichgewichtskurve sowie einen pH2O aufweist, welche mit den entsprechenden Werten der Kalibrier- oder Konditionierflüssigkeit - innerhalb der gewünschten Meßgenauigkeit für die zu messenden Probenbestandteile - übereinstimmen.

Weiter ist es von Vorteil, wenn im interessierenden Meßbereich die Ionenstärke der Qualitätskontrollflüssigkeit bei einem vorgegebenen pH-Wert - innerhalb der gewünschten Meßgenauigkeit für die zu messenden Probenbestandteile - mit jener der Kalibrier- oder Konditionierflüssigkeit übereinstimmt, bzw. wenn der osmotische Druck der Qualitätskontrollflüssigkeit mit jenem der Kalibrier- oder Konditionierflüssigkeit übereinstimmt.

Eine Qualitätskontrollflüssigkeit zur Durchführung des erfindungsgemäßen Verfahrens zeichnet sich dadurch aus, daß die Qualitätskontrollflüssigkeit im Hinblick auf jene chemischen oder physikalischen Parameter, welche die Kennlinie der einzelnen Sensoren bei einer vorangehenden Kalibrierung oder Konditionierung beeinflussen, mit der Kalibrier- oder Konditionierflüssigkeit - innerhalb der gewünschten Meßgenauigkeit für die zu messenden Probenbestandteile - übereinstimmt.

Die Einmal-Kassetten können mit einer beliebigen Kombination folgender Sensoren bestückt sein, wobei das erfindungsgemäße Verfahren zur Anwendung kommen kann:

### pH-Sensoren:

Optische Sensoren zur Bestimmung des pH-Wertes enthalten zumeist einen kovalent, elektrostatisch oder adsorptiv in einer ionenpermeablen, zumeist hydrophilen Polymerschicht immobilisierten pH-Indikator in Form einer organischen Säure oder Base. Die immobilisierten pH-Indikatoren können durch Austausch von Ionen (z.B., H+ bzw. OH-) über ionenpermeable Materialien in zumindest indirekten Kontakt mit einer Probe gebracht werden (M.J.P. Leiner und O.S.Wolfbeis, "Fiber Optic pH-Sensors", in O.S. Wolfbeis "Fiber Optic Chemical Sensors and Biosensors", CRC-Press, Boca Raton, 1991, Chapter 8). In Abhängigkeit des pH Wertes des Meßgutes (pH = -log(aH+)) stellt sich ein thermodynamisches Gleichgewicht zwischen protonierten und deprotonierten Formen des pH-Indikators ein. Aus dem mit optischen Methoden meßbaren Konzentrationsverhältnis beider Formen kann auf den pH-Wert des Meßgutes rückgeschlossen werden.

Als elektrochemische Sensoren zur Bestimmung des pH-Wertes kommen beispielsweise Glaselektroden, Flüssigmembranelektroden, Antimonelektroden, Feldeffektransistoren (ISFET), Festkörpersysteme (z.B. Edelmetall/Edelmetalloxidsysteme wie Ir/IrO2 und Pd/PdO2), und Redoxsysteme in Frage.

### CO2-Sensoren:

Optische Sensoren zur Bestimmung des CO2-Partialdruckes eines flüssigen Meßgutes bestehen zumeist aus einem schichtförmig ausgebildeten Reaktionsraum und einem ionenimpermeablen, gas-permeablen, den Reaktionsraum vom Meßgut trennenden Material. Der Reaktionsraum ist häufig identisch mit dem indikatortragenden Material eines optischen pH-Sensors. Zudem befinden sich üblicherweise im Reaktionsraum eine oder mehrere pH-Puffersubstanzen, beispielsweise Carbonate, Phosphate, und/oder in wäßrigen Medien sauer oder basisch reagierende organische Verbindungen. Bei gegebener Temperatur, Wassergehalt, Zusammensetzung der puffernden Substanzen, ist der pH-Wert des Reaktionsraumes in charakteristischer Weise abhängig vom pCO2 des Meßgutes.

Elektrochemische Sensoren zur Bestimmung des CO2-Partialdruckes haben häufig einen prinzipiell ähnlichen Schichtaufbau. Anstelle eines optischen pH-Indikators wird eine der oben beschriebenen pH-sensitiven Elektroden zur Messung des pH-Wertes des Reaktionsraumes herangezogen.

Da gaspermeable, ionenimpermeable Trennmaterialien, auch Wasser durch isotherme Destillation durchlassen, erfolgt neben dem Gasaustausch zwischen Meßgutraum und Reaktionsraum auch ein Austausch von Wasser. Die Aufnahme bzw. Abgabe von Wasser, erfolgt, wenn der Wasserdampfpartialdruck bzw. osmotische Druck des Reaktionsraumes sich von jenem des Mediums im Meßgutraum unterscheidet. Der Austauschprozeß kommt erst dann in einen Gleichgewichtszustand, wenn der Wasserdampfpartialdruck und/oder osmotische Druck beider Räume den gleichen Wert angenommen hat.

### O2-Sensoren:

Optische Sensoren zur Bestimmung des O2-Patialdruckes eines flüssigen oder gasförmigen Meßgutes enthalten zumeist einen kovalent, elektrostatisch oder adsorptiv in einer, vorzugsweise ionenimpermeablen Polymerschicht immobilisierten oder gelösten optischen O2-Indikator. Zur Vermeidung optischer Interferenzen mit dem Meßgut wird häufig eine zweite, mit Farbpigmenten versetzte, vorzugsweise ionenimpermeable Polymerschicht zur optischen Abkopplung des Meßgutraumes von der indikatortragenden Polymerschicht verwendet.

Elektrochemische Sensoren zur Bestimmung des O2-Patialdruckes eines flüssigen oder gasförmigen Meßgutes sind zumeist amperometrische Elektroden. Die eigentliche Elektrodenanordnung ist häufig durch ein ionenimpermeables, gaspermeables Trennmaterial vom Meßgutraum getrennt.

### Ionensensoren:

In der wissenschaftlichen Literatur werden eine Reihe unterschiedlich aufgebauter optischer Erkennungssysteme zur Bestimmung der Konzentration (bzw. Aktivitäten) anorganischer Kationen oder Anionen (NH4+, Li+, K+, Na+, Mg++, Ca++, Cl-) beschrieben. In Abhängigkeit des Erkennungssystems liegen die chemischen Komponenten in ionenpermeablen, häufig schichtförmig aufgebauten Polymermaterialien vor.

Elektrochemische Sensoren zur Bestimmung der Konzentration bzw. Aktivitäten der genannten anorganischen Kationen oder Anionen sind zumeist potentiometrische Meßketten. Das Erkennungssystem beruht auf einem Ionophor, welcher physikalisch gelöst in einem Polymermaterial vorliegt.

### Substratsensoren:

In der wissenschaftlichen Literatur beschriebene optische und elektrochemische Sensoren zur Bestimmung der Konzentration von mit Enzymen reagierenden biologischen Substanzen wie beispielsweise Glucose, Lactat, Harnstoff, beruhen auf Erkennungssystemen, welche zumeist eine spezifische biochemische Reaktion mit der zu bestimmenden Substanz (Substrat) eingehen. Parallel zum enzymatischen Substratabbau werden weitere Stoffe, beispielsweise O2, H2O2, NH4+, H+ verbraucht und/oder gebildet. Letztere können mit den oben beschriebenen Sensoren bestimmt werden.

So wird häufig zur Bestimmung der Glucosekonzentration ein oben beschriebener optischer oder elektrochemischer O2-Sensor verwendet, welcher zusätzlich ein Enzym enthält. Mittels der O2-Messung kann die Konzentration der Glucose im Meßgut ermittelt werden.

### Referenzelektroden:

Im Gegensatz zu optischen Sensoren benötigen elektrochemische Meßanordnungen ein Referenzelement. Bei potentiometrischen Elektroden ist die Referenzelektrode häufig im Meßgutraum, räumlich getrennt von den eigentlichen Meßelektrode angeordnet. In einer beispielhaften Ausführungsvariante besteht die potentiometrische Referenzelektrode aus einer Metallelektrode (z.B. Ag), welche über ein ionenpermeables Material in indirektem Kontakt mit dem Meßgutraum steht. Das ionenpermeable Material enthält die für den potentialbildenden Prozeß erforderlichen ionischen Komponenten (z.B. AgCl/KCl).

Eine zur simultanen Kalibration von mehreren Sensoren in einer Einmal-Kassette geeignete Flüssigkeit enthält die zu bestimmenden ionischen bzw. Gaskomponenten mit bekannten Konzentrationen bzw. Partialdrücken, welche einem Erwartungswert der Meßgröße in der jeweiligen Meßsituation entsprechen. Eine derartig zusammengesetzte Flüssigkeit kann grundsätzlich auch zur Konditionierung der Sensoren verwendet werden. Da die Löslichkeiten der Gaskomponenten (O2, CO2) in wäßrigen Flüssigkeiten stark temperaturabhängig sind, und zudem ein diffusionskontrollierter, temperaturabhängiger Gasaustausch mit den Kunststoffmaterialien der Einmal-Kassette erfolgt, sind, insbesondere beim thermostatisieren der Einmal-Kassette von der Lagertemperatur (beispielsweise 0-35°C) auf die Meßtemperatur (37°C), die Gaspartialdrücke der Kalibrierflüssigkeit, zum Zeitpunkt der Kalibration nicht mit hinreichender Genauigkeit bekannt.

Eine Möglichkeit genauere bekannte Gaspartialdrücke zum Zeitpunkt der Kalibration zu realisieren ist die Verwendung eines mit Kalibriergas beaufschlagten flüssigen Kalibriermediums in einem separaten, vorzugsweise in der Einmal-Kassette vorliegenden gasimpermeablen Gebinde (WO 92/01928).

Soll das Vorliegen zweier Flüssigkeiten (Konditionierflüssigkeit und Kalibrierflüssigkeit) in einer Einmal-Kassette vermieden werden, so müssen die Sensoren bis zur Kalibration trocken in einer Einmal-Kassette vorliegen. Durch den Kontakt mit flüssigen Medien (Kalibrierflüssigkeit, Kontrollflüssigkeit, wäßriges Meßgut) nehmen trocken gelagerte Sensoren langsam Wasser auf (Hydratation, "wet-up", Konditionierung). Die Kinetik der Hydratation ist von unterschiedlichen Faktoren abhängig (z.B. Temperatur, pH, physikochemische Materialeigenschaften). Da die Sensorkennlinie vom Hydratationszustand der jeweiligen Sensoren abhängt, verändert sich diese stetig mit ungleichförmiger Geschwindigkeit. Die WO 92/01928 offenbart rechnergestützte Methoden zur Kompensation dieser, sich monoton durch Hydratationsprozesse verändernder Sensorsignale.

Mit dem erfindungsgemäßen Verfahren lassen sich auch trocken gelagerte Sensoren einer Qualitätskontrolle unterziehen, wobei die Verfahrensschritte gemäß Anspruch 5 zu beachten sind.

Zum Zeitpunkt der Kalibration wesentlich genauer bekannte Gaspartaldrücke können beispielsweise dadurch realisiert werden, daß die in Kontakt mit einem flüssigen Konditioniermedium gelagerten Sensoren mit einem, vorzugsweise im portablem Analysator vorhandenen Kalibriergas bekannter Gaszusammensetzung beaufschlagt werden. Bedingt durch den vorgelagerten, hinreichend langen Kontakt der Sensoren mit der Konditionnierflüssigkeit, deren in den ionenpermeablen Materialien eines allfällig vorhandenen pH-Sensors verbleibenden Reste, beaufschlagt mit einem CO2-hältigen Kalibriergas, ein Kalibriermedium für den pH-Sensor ergeben, befinden sich die zwischen den Sensoren und dem flüssigen Kalibriermedium durch Diffusion austauschbaren Komponenten im chemischen Gleichgewicht.

Das erfindungsgemäße Verfahren für Sensoren, welche in Kontakt mit einem flüssigen Konditioniermedium gelagert werden, ist durch die Verfahrensschritte gemäß Anspruch 1 gekennzeichnet.

Durch die variable Konzentrationen der, zwischen Kalibierlüssigkeit, Kontrollflüssigkeit, oder Meßgut und einer Referenzelektrode austauschbaren Elektrolyte, bildet sich an der Phasengrenzfläche potentiometrischer Referenzelektroden ein Diffusionspotential aus. Sind die entsprechenden Elektrolytkonzentrationen in beiden Phasen bekannt, so kann das Diffusionspotential berechnet, und damit seine Auswirkung auf das Meßergebnis berücksichtigt werden. Bei gegebenen Materialien hängt die Kinetik des Austauschprozesse von der Temperatur, den Konzentrationsgradienten der Elektrolyte, und den physikalischen Eigenschaften der verwendeten Materialien ab. Zu einem bestimmten Zeitpunkt nach dem Kontakt mit salzhaltigen Flüssigkeiten, kann das Diffusionspotential in Abhängigkeit von der Temperatur und Dauer des Kontaktes berechnet werden.

Im Zusammenhang mit den verwendeten Sensoren ist darauf hinzuweisen, daß niedermolekulare, vorzugsweise elektrisch neutrale Moleküle langsam sowohl durch ionenpermeable als auch ionenimpermeable Sensormaterialien diffundieren und durch Verändern der chemischen und/oder physikalischen Materialeigenschaften bzw. Wechselwirkung mit chemischen Erkennungsystemen die Sensorkennlinie optischer und elektrochemischer Sensoren beeinflussen können.

Vorzugsweise niedermolekulare elektrisch geladene Moleküle (Ionen), können zumeist ungehindert durch hydrophyle Polymermaterialien (Hydrogele) diffundieren und die Sensorkennlinie entsprechend aufgebauter Sensoren (z.B. optische pH-Sensoren, optische Ionensensoren, optische und elektrochemische Substratsensoren) verändern.

Die Austauschprozesse kommen erst dann in einen Gleichgewichtszustand, wenn sich die chemischen Potentiale der auszutauschenden Stoffe ausgeglichen haben. Es ist zwar grundsätzlich möglich aufgrund der bekannten Dauer und Temperatur des Kontaktes mit flüssigen Medien, rechnerisch auf die zu einem gegebenen Zeitpunkt vorliegende Sensorkennlinie zu schließen, jedoch ist dieses Verfahren sehr ungenau, insbesondere beim sequentiellen Kontakt mit unterschiedlich zusammengesetzten Flüssigkeiten (Kalibrierflüssigkeit, Kontrollflüssigkeit, Meßgut). Durch die Verwendung der erfindungsgemäßen Qualitätskontrollflüssigkeit treten die genannten Nachteile nicht auf.

Eine ideale Qualitätskontrollflüssigkeit für Sensoren in einer Einmal-Kassette wäre in ihrer Zusammensetzung identisch jener der Kalibrierflüssigkeit (bei substantiell trocken gelagerten Sensoren) bzw. identisch den in den Materialien der Sensoren verbleibenden Resten der Konditionierflüssigkeit (bei naß gelagerten Sensoren). Die Anforderungen an eine erfindungsgemäße Qualitätskontrollflüssigkeit müssen jedoch nur den Merkmalen der Patentansprüche genügen, da
1.) Geringe Beimengungen gewisser Additive keinen signifikanten Einfluß auf die Meßeigenschaften der Sensoren haben,
2.) Bei Vorliegen von Substratsensoren (z.B. Glucosesensor) die Kalibrierflüssigkeit beispielsweise kein Substrat enthält, während die Qualitätskontrollflüssigkeit eine dem Erwartungswert der Substratkonzentration entsprechende Substratkonzentration enthalten soll,
3.) Zur Kontrolle der Ionensensoren unterschiedliche relative Konzentrationen an Kationen (Na+, K+, ..) möglich sind und lediglich eine gleiche Gesamtionenstärke notwendig ist.

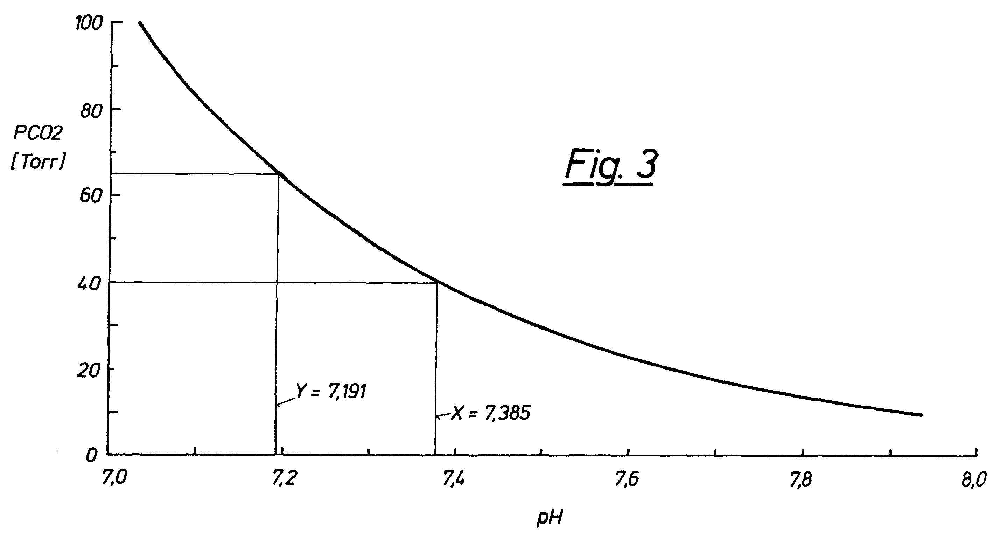

Die dem erfindungsgemäßen Verfahren genügende Kontrollflüssigkeit ist vielmehr durch folgende Merkmale gekennzeichnet:
1. im interessierenden Meßbereich (pCO2 10-120 Torr, pH 6.8-8.0) und bei gegebener Meßtemperatur (20-40°C), ist die CO2/pH-Gleichgewichtskurve (pH=f(pCO2,T, Konzentrationen der pH-Pufferkomponenten, Ionenstärke)) (siehe auch "The Acid-Base Status of the Blood", Siggaard-Anderson, Munksgaard, Copenhagen 1974; oder "pH-Wert Berechnung", C. Bliefert, Verlag Chemie Weinheim, New York 1978) der Qualitätskontrollflüssigkeit mit jener der Kalibrierflüssigkeit bzw. der Konditionierflüssigkeit innerhalb der gewünschten Meßgenauigkeit ident (siehe Fig. 3).
2. Insbesondere bei Verwendung von optischen pH- und Ionensensoren, soll die Ionenstärke der Kontrollflüssigkeit, bei einem gegebenen pH-Wert im interessierenden Meßbereich mit jener der Kontrollflüssigkeit bzw. der Konditionierflüssigkeit übereinstimmen.
3. Die Qualitätskontrollflüssigkeit kann beispielsweise einen beliebigen pCO2 und auch pO2 im interessierenden Meßbereich aufweisen. Die Gaspartialdrücke an pCO2 und pO2 können verschieden von jenen der Kalibrierflüssigkeit bzw. der Konditionierflüssigkeit und des Kalibriergases sein.

Zur Durchführung des erfindungsgemäßen Verfahrens geeignete Einmal-Kassetten sind beispielsweise aus der eingangs genannten EP 0 460 343 B1 bekannt.

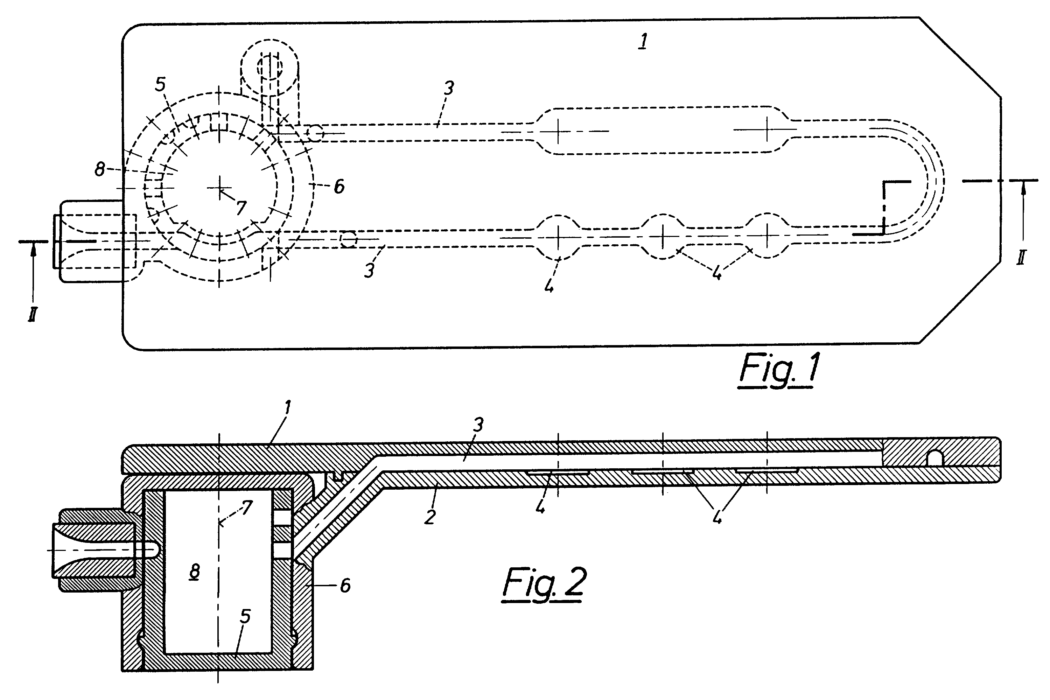

Weiters eignet sich dafür auch die in den Fig. 1 und 2 der vorliegenden Erfindung skizzierte Ausführungsvariante einer derartigen Einmal-Kassette, wobei Fig. 1 eine Draufsicht auf die Kassette und Fig. 2 einen Schnitt entlang der Linie II-II in Fig. 1 zeigt.

Die Kassette besteht im wesentlichen aus einem Gehäuseoberteil 1 und einem Gehäuseunterteil 2, nach deren Zusammenbau ein U-Förmig verlaufender Probenkanal 3 entsteht. Im Probenkanal 3 sind Sensoren 4, beispielsweise zur Messung von pH, CO2 und O2 angeordnet. Zur Steuerung der verschiedenen Medien ist ein drehbares Ventil 5 in einer Anformung 6 des Gehäuseunterteils 2 angeordnet, dessen Achse 7 normal auf die durch die beiden Schenkel des Probenkanals aufgespannte Ebene steht. Im Inneren des als Hohlzylinder ausgeführten Ventils 5 ist ein Abfallbehälter 8 zur Aufnahme der Konditionierflüssigkeiten der Kalibriermedien, der Qualitätskontrollflüssigkeit sowie ggf. der Probe vorgesehen. Es ist auch möglich, bei trocken gelagerten Sensoren, im Abfallbehälter 8 des Ventiles 5 vorher die Kalibrierflüssigkeit zu lagern.

Als Sensoren zur Durchführung des erfindungsgemäßen Verfahrens können optische oder elektrochemische Sensoren für pH, pO2, pCO2, Li+, Na+, K+, Mg ++, Ca++, Cl-, Glucose, Lactat, Harnstoff (BUN), Harnsäure, Kreatinin, Cholesterin verwendet werden.

Bei der Zusammensetzung einer allfälligen Konditionierflüssigkeit sowie einer Kalibrierflüssigkeit zur nachfolgenden Durchführung des erfindungsgemäßen Verfahrens zur Qualitätskontrolle ist folgendes zu beachten:

Bei Vorliegen eines pH-Meßelementes: Die 1-Punkt-Kalibration von pH-Meßelementen für biologische Flüssigkeiten (beispielsweise Vollblut) erfolgt vorteilhafterweise bei pH-Werten im physiologisch normalen pH-Bereich (ca. 7.35-7.45). Geeignete chemische Komponenten wäßriger Flüssigkeiten zur Realisierung dieser Werte sind beispielsweise Phosphate, organische zwitterionische Verbindung, beispielsweise HEPES, MOPS, TES (beschrieben in Good et al. Biochemistry 5, 467-477, 1996), sowie organische N-haltige Basen, beispielsweise TRIS.

Beispielhafte Flüssigkeit: Wasser, welches 0.008695 (mol/kg) KH2PO4 und 0.03043 (mol/kg) Na2PO4 in gelöster Form enthält, weist bei 37°C und in Abwesenheit von CO2 eine Ionenstärke von 0,100(mol/kg) und einen pH von 7.385 auf.

Einstellung der Ionenstärke: Da insbesondere die Sensorkennlinie optischer pH-Meßelemente von der Ionenstärke flüssiger Meßmedien abhängt, ist es von besonderem Vorteil, beim Konditionierschritt bzw. Kalibrierschritt eine, der Art des Meßgutes angepaßte Ionenstärke zu gewährleisten. Werte der Ionenstärke von Vollblut liegen bei ca. 0.130-0.170 mol/l.

Zur Anpassung der Ionenstärke können der Konditionierflüssigkeit bzw. Kalibrierflüssigkeit in wäßriger Lösung neutral reagierende Salze zugesetzt werden. Geeignet sind alle in wäßriger Umgebung löslichen, salzartigen Verbindung von starken Säuren mit starken Laugen, beispielsweise NaCl, KCl, LiCl, Na2SO4, NaN03, oder Salze schwacher Säuren bzw. schwacher Basen (z.B. CH3COOLi) insofern diese im interessierenden pH-Meßberiech dissoziiert bzw. protoniert vorliegen. Da solche Anionen bzw. Kationen auch Gegenionen von pH-Pufferkomponenten sein können, müssen die Konzentrationen aller Komponenten entsprechend angeglichen werden.

Bei Vorliegen von pH und CO2 Meßelementen: Zur simultanen 1-Punkt-Kalibration eines, in einer Einmal-Kassette vorhandenen C02-Sensors für die Blutgasanalyse ist es von Vorteil wenn die Flüssigkeit mit einem CO2-Partialdruck im physiologischen Normalbereich beaufschlagt wird (ca. 35-45 Torr). Durch die Beaufschlagung mit 40 Torr CO2 verändert sich der oben angeführte pH-Wert auf 6.961 bei einer Ionenstärke von 0,092 (mol/l). Ein Zusatz von 0,024 (mol/l) NaHCO3 bewirkt, daß der pH-Wert wieder 7.385 bei einer Ionenstärke von 0.124 mol/l beträgt.

Bei Verwendung von O2 Meßelementen: Zur simultanen 1-Punkt-Kalibration eines, in der Einmal-Kassette vorhandenen O2-Sensors ist es von Vorteil, wenn das Kalibriermedium einen pO2 im physiologischen Normalbereich aufweist (ca. 90-110 Torr). Die Ionen wäßriger Medien beeinflussen diese Sensoren im allgemeinen nicht, sehr wohl jedoch der pH2O bzw. der osmotischen Druck.

Bei Verwendung von Ionensensoren: Zur simultanen 1-Punkt-Kalibration von in einer Einmal-Kassette zusätzlich vorhandener Ionensensoren (beispielsweise für Li+, Na+, K+, Mg++, Ca++, Cl-), ist es von besonderem Vorteil die Konzentrationen (bzw. Aktivitäten) der entsprechenden Kationen und Anionen auf normale Werte des Meßgutes einzustellen. Normalwerte von Vollblut liegen beispielsweise im Bereich von 135-150 (mmol/l) für Na+, 3.5-5.0 (mmol/l) für K+, 1.0-1.4 (mmol/l) für Ca++, 98.0-119,0 (mmol/l) für Cl-.

### Beispielhafte Kalibrier- und Konditioniermedien:

Liegen beispielsweise in der Einmal-Kassette Sensoren zur Bestimmung des pH-Wertes, des pCO2 und pO2, sowie der K+, Na+, und Cl-Konzentrationen von Vollblut vor, so erfüllt eine wäßrige Flüssigkeit, beaufschlagt mit 40 Torr CO2, 90 Torr O2, bei einer Temperatur von 37°C der Zusammensetzung

| Komponente [mol/l] | | Na+ [mol/l] | K+ [mol/l] | Cl-[mol/l] | Ionenstärke [mol/l] |
|---|---|---|---|---|---|
| NaH2PO4 | 0.0028 | 0.0028 | | | 0.0028 |
| Na2HPO4 | 0.0112 | 0.0224 | | | 0.0336 |
| NaHCO3 | 0.0243 | 0.0243 | | | 0.0243 |
| NaCl | 0.0960 | 0.0960 | | 0.0960 | 0.0960 |
| KCl | 0.0040 | | 0.0040 | 0.0040 | 0.0040 |
| | Summen: | 0.1455 | 0.0040 | 0.1000 | 0.1607 |

die oben genannten Anforderungen. Die Flüssigkeit weist einen pH-Wert von 7.385 (siehe Fig. 3, Punkt X), eine Ionenstärke von 160 mmol/l, eine Na+ Konzentration von 145 (mmol/l), eine K+ Konzentration von 4(mmol/l) und eine Cl-Konzentration von 100(mmol/l) auf.

Die CO2/pH Gleichgewichtskurve dieser Flüssigkeit ist in Fig. 3 angegeben.

Tenside: Durch längeren Kontakt der Sensoren mit einer Konditionierflüssigkeit nimmt die Blasenbildung zu. In der Meßanordnung befindliche Blasen erschweren das Ersetzen von Flüssigkeiten durch gasförmige Medien. Zur Vermeidung von Blasenbildung und zur Erhöhung der Benetzbarkeit ist es von Vorteil geringe Mengen an Tensiden zuzusetzen (Beispiele: Triton X100: Du Pont Company, 549 Albany Str.Boston, MA 02118, USA; Dehydron 241, Dehydrol 100: Henkel Corporation, 300 Brookside Ave. Ambier, PA 19002, USA).

Biozide: Häufig bewirken biologische Aktivitäten eine Veränderung der Zusammensetzung wäßriger Flüssigkeiten, insbesondere des pH-Wertes. Infolge dieser Aktivitäten ist der pH-Wert bei der Kalibration bzw. Kontrollmessung nicht hinreichend genau bekannt. Daher ist es vorteilhaft, den Flüssigkeiten geringe Mengen an Bioziden zuzusetzen (Beispiele: NaN3, Mergal K9N: Manufactured by Riedel de Haan AG, marketed through Hoechst Austria AG, A-1121 Vienna; Proclin 300, Proclin 150: Supelco, Inc. Supelco Park, Bellefonte, PA 16823, USA; Bronidox L: Henkel Corporation, 1301 Jefferson Str. Hoboken, NJ 07030 USA; Nuosept C: Huls America, Inc., Turner Place, P.O. Box 365, Piscataway, NJ 08855).

Gaslöslichkeit: Weiters können flüssigen Kalibriermedien Substanzen zur Erhöhung der Gaslöslichkeit zugesetzt werden (beispielsweise Fluorocarbone zur Erhöhung der Sauerstofflöslichkeit).

Ein Beispiel für die Zusammensetzung einer Qualitätskontrollflüssigkeit zur Durchführung des erfindungsgemäßen Verfahrens sei im folgenden dargelegt:

Eine geeignete Qualitätskontrollflüssigkeit enthält die mit den Sensoren einer Einmal-Kassette zu bestimmenden Parameter in Konzentrationen bzw. mit Partialdrücken, welche den jeweiligen Erwartungswerten des Meßgutes entsprechen. Vorteilhafterweise sind die Werte dieser Parameter identisch mit denjenigen der Kalibrierflüssigkeit.

Die Kontrollflüssigkeit ist mit einem Gas bekannter Zusammensetzung an O2 und CO2 beaufschlagt und wird bis zum Gebrauch in einem gas- und wasserimpermeablen Behältnis (vorzugsweise einer Glasampulle) aufbewahrt. Die relative Zusammensetzung der beaufschlagten Gasmischung kann von jener der Konditionierflüssigkeit bzw. Kalibrierflüssigkeit abweichen.

Die oben (siehe Tabelle Seite 17) beispielsweise angeführte Konditionier- bzw. Kalibrierflüssigkeit eignet sich auch als Qualitätskontrollflüssigkeit. Da sie identisch mit der Konditionier- bzw. Kalibrierflüssigkeit ist, stimmt sie auch in allen physikalischen und chemischen Parametern mit der beispielhaft angeführten Konditionier- und Kalibrierflüssigkeit überein. Es sei darauf hingewiesen, daß die Qualitätskontrollflüssigkeit nur mit jenen physikalischen und/oder chemischen Parametern übereinstimmen muß, welche die Kennlinie der Sensoren innerhalb der gewünschten Meßgenauigkeit für die zu messenden Probenbestandteile beeinflussen.

Es ist daher ausreichend, wenn die Qualitätskontrollflüssigkeit zur Durchführung des erfindungsgemäßen Verfahrens hinsichtlich der CO2/pH-Gleichgewichtskurve und der Ionenstärke mit der Kalibrierflüssigkeit bzw. Konditionierflüssigkeit übereinstimmt.

Bei Vorliegen von Sensoren mit ionenimpermeablen Trennmembranen soll zudem der Wasserdampfpartialdruck der Kontrollflüssigkeit mit jenen der Kalibrier- bzw. Konditionierflüssigkeit übereinstimmen.

Bei Verwendung weiterer chemischer Zusätze müssen die Einflüsse dieser chemischen Parameter auf die angeführten physikalischen Größen berücksichtigt werden.

Insbesondere bei biologischem Meßgut (z.B. Vollblut) ist es häufig erforderlich, neben den Kontrollwerten im physiologischen Normalbereich der jeweiligen Meßgrößen auch Kontrollwerte oberhalb und unterhalb dieser Wertebereiche zur Verfügung zu stellen.

Beaufschlagt man beispielsweise die oben angeführte Qualitätskontrollflüssigkeit bei 37°C mit 65 Torr CO2 und 60 Torr O2, so ändert sich die Zusammensetzung der pH-Pufferkomponenten, während die Konzentrationen der Kationen und Anionen von in wäßriger Umgebung löslichen, salzartigen Verbindungen starker Säuren mit starken Laugen (Na+, K+, Cl-) gleich bleibt:

| Komponente [mol/l] | | Na+ [mol/l] | K+ [mol/l] | Cl-[mol/l] | Ionenstärke [mol/l] |
|---|---|---|---|---|---|
| NaH2PO4 | 0.0039 | 0.0039 | | | 0.0039 |
| Na2HPO4 | 0.0100 | 0.0200 | | | 0.0300 |
| NaHCO3 | 0.0255 | 0.0255 | | | 0.0255 |
| NaCl | 0.0960 | 0.0960 | | 0.0960 | 0.0960 |
| KCl | 0.0040 | | 0.0040 | 0.0040 | 0.0040 |
| | Summen: | 0.1454 | 0.0040 | 0.1000 | 0.1594 |

Der pH-Wert beträgt 7.191 (siehe Fig. 3, Punkt Y). Die CO2/Gleichgewichtskurve ist unverändert. Innerhalb der gewünschten Meßgenauigkeit für die zu messenden Probenbestandteile bleibt somit die Ionenstärke gleich.

Sollen zusätzlich Kontrollwerte für Ionensensoren außerhalb der jeweiligen physiologischen Normalbereiche realisiert werden, so können die Konzentrationen der zugesetzten Neutralsalze verändert werden. Da eine Veränderung der Konzentrationen der Neutralsalze die Ionenstärke beeinflußt, ist es zwecks Angleichung der Ionenstärke an diejenige der Konditioner- bzw. Kalibrierflüssigkeit beispielsweise möglich, ein weiteres Neutralsalz zuzugeben dessen anionische- bzw. kationische Bestandteile verschieden von den zu messenden Probenparametern sind (z.B. Li2SO4, LiNO3, CH3COOLi).

| Komponente [mol/l] | | Na+ [mol/l] | K+ [mol/l] | Cl-[mol/l] | Ionenstärke [mol/l] |
|---|---|---|---|---|---|
| NaH2PO4 | 0.0039 | 0.0039 | | | 0.0039 |
| Na2HPO4 | 0.0100 | 0.0200 | | | 0.0300 |
| NaHCO3 | 0.0255 | 0.0255 | | | 0.0255 |
| NaCl | 0.0660 | 0.0660 | | 0.0660 | 0.0660 |
| KCl | 0.0060 | | 0.0060 | 0.0060 | 0.0060 |
| LiNO3 | 0.0280 | | | | 0.0280 |
| | Summen: | 0.1154 | 0.0060 | 0.0720 | 0.1594 |

Die Flüssigkeit weist einen pH-Wert von 7.191 auf. Sämtliche, beispielhaft angeführten zu messenden Probenparameter liegen nun außerhalb des physiologischen Normalbereiches von Vollblut. Trotzdem bleibt die CO2/pH Gleichgewichtskurve sowie die Ionenstärke unverändert (Fig. 3, Punkt Y).

Der Verfahrensablauf bei Verwendung naßgelagerter Sensoren (z.B. EP 0 460 343 B1) stellt sich wie folgt dar:

### - - VORBEREITUNG DER KASSETTE & KONDITIONIERUNG - -

1. Ein- oder mehrfaches Befüllen des Probenraumes (Probenkanals) mit Konditionierflüssigkeit. Verschluß des Meßraums.
2. Verpackung der Einmal-Kassette in einem gas- und wasserimpermeablen Gebinde. Lagerung bis zum Gebrauch. (Die Konditionierung der Sensoren kann nach dem Befüllen des Meßraumes oder während der Lagerung erfolgen).
3. Entnahme der Kassette aus der Verpackung, Einlesen der Sensorkenndaten in das Meßinstrument und Einlegen der Kassette in den portablen Analysator.

### - - KALIBRATIONSMESSUNG - -

4. Verdrängen der im Probenraum (Probenkanal) befindlichen Konditionierflüssigkeit mittels Kalibriergas in ein (beispielsweise in der Kassette integriertes) Waste, wobei Reste der Konditionierflüssigkeit in den ionenpermeablen Schichten der Sensoren verbleiben, und diese Reste sowie die Sensormaterialien mit dem Kalibriergas beaufschlagt werden.
5. Simultane Erhebung der Kalibrationssignale der verschiedenen Sensoren.

### - - KONTROLLMESSUNG - -

6. Ersetzen des Kalibriergases mit einer erfindungsgemäßen Qualitätskontrollflüssigkeit, welche sich in einem Gas- und Wasser- impermeablen (Glas)gebinde befindet.
7. Simultane Erhebung der Kontrollsignale der verschiedenen Sensoren.
8. Ermittlung der Ist-Kontrollwerte (pH, Konzentrationen/Gaspartialdrucke), unter Berücksichtigung der eingelesenen Sensorkenndaten, der erhobenen Kalibrationssignale (Schritt 5) und der erhobenen Kontrollsignale (Schritt 7). Anzeige der Ist-Kontrollwerte.
9. Vergleich der ermittelten Ist-Kontrollwerte mit den bekannten Soll-Kontrollwerten, wobei aus den Differenzen dieser Werte Rückschlüsse auf die Genauigkeit und Zuverlässigkeit des portablen Analysators bzw. der Kassette gezogen werden.

### - - PROBENMESSUNG - -

10. Verdrängen der im Probenraum (Probenkanal) befindlichen Qualitätskontrollflüssigkeit mittels eines Kalibriergases in ein (beispielsweise in der Kassette integriertes) Waste.
11. Ersetzen des Kalibrationsgases durch das Meßgut (z. B. Blut).
12. Simultane Erhebung der Meßgutsignale der verschiedenen Sensoren.
13. Ermittlung der Meßgrößen (pH, Konzentrationen/Gaspartialdrucke), unter Berücksichtigung der eingelesenen Sensorkenndaten, der erhobenen Kalibrationssignale (Schritt 5) und der erhobenen Meßgutsignale (Schritt 12). Anzeige der Meßgrößen.

Der Verfahrensablauf bei Verwendung trocken gelagerter Sensoren (z. B. WO 92/01928) stellt sich wie folgt dar:

### - - VORBEREITUNG DER KASSETTE - -

1. Befüllen eines, vorzugsweise in der Einmal-Kassette befindlichen gas- und wasserimpermeablen Reservoirs mit Kalibrierflüssigkeit. Verschluß des Reservoirs.
2. Verpackung der Kassette in einem gas- und wasserimpermeablen Gebinde. Lagerung bis zum Gebrauch.
3. Entnahme der Kassette aus der Verpackung, Einlesen der Sensorkenndaten in das Meßinstrument und Einlegen der Kassette in den portablen Analysator.

### - - KALIBRATIONSMESSUNG - -

4. Herstellung einer Verbindung zwischen Reservoir und Probenraum (Probenkanal). Überführung der im Reservoir befindlichen Kalibrierflüssigkeit in den Probenraum (Probenkanal).
5. Simultane Erhebung der Kalibrationssignale der verschiedenen Sensoren unter Berücksichtigung des Zeitpunktes der Erhebung der Signale relativ zum Zeitpunkt des Erstkontaktes mit der Kalibrationsflüssigkeit.

### - - KONTROLLMESSUNG - -

6. Ersetzen der im Probenraum (Probenkanal) befindlichen Kalibrierflüssigkeit mit einem gasförmigen Medium bekannter Zusammensetzung (kann auch Raumluft sein). Ersetzen des im Probenraum (Probenkanal) befindlichen gasförmigen Mediums mit einer erfindungsgemäßen Qualitätskontrollflüssigkeit welche sich in einem gas- und wasserimpermeablen (Glas)gebinde befindet.
7. Simultane Erhebung der Kontrollsignale der verschiedenen Sensoren unter Berücksichtigung des Zeitpunktes der Erhebung der Signale relativ zum Zeitpunkt des Erstkontaktes mit der Kalibrationsflüssigkeit.
8. Ermittlung der Ist-Kontrollwerte (pH, Konzentrationen/Gaspartialdrucke), unter Berücksichtigung der eingelesenen Sensorkenndaten, des Zeitpunktes der Erhebung der Meßgutsignale relativ zum Zeitpunkt des Erstkontaktes mit der Kalibrationsflüssigkeit, der erhobenen Kalibrationssignale (Schritt 5) und der erhobenen Kontrollsignale (Schritt 7).
9. Vergleich der ermittelten Ist-Kontrollwerte mit den bekannten Soll-Kontrollwerten, wobei aus den Differenzen dieser Werte Rückschlüsse auf die Genauigkeit und Zuverlässigkeit des portablen Analysators bzw. der Kassette gezogen werden.

### - - PROBENMESSUNG - -

10. Ersetzen der im Probenraum (Probenkanal) befindlichen Qualitätskontrollflüssigkeit mittels eines gasförmigen Mediums bekannter Zusammensetzung (z.B. Raumluft), wobei die Kontrollflüssigkeit in ein (beispielsweise in der Kassette integriertes) Waste überführt wird.
11. Ersetzen des im Probenraum (Probenkanal) befindlichen gasförmigen Mediums durch das Meßgut (Blut).
12. Simultane Erhebung der Meßgutsignale der verschiedenen Sensoren unter Berücksichtigung des Zeitpunktes der Erhebung der Signale relativ zum Zeitpunkt des Erstkontaktes mit der Kalibrationsflüssigkeit.
13. Ermittlung und Anzeige der Meßgrößen (pH, Konzentrationen/Gaspartialdrucke), unter Berücksichtigung der eingelesenen Sensorkenndaten, des Zeitpunktes der Erhebung der Meßgutsignale relativ zum Zeitpunkt des Erstkontaktes mit der Kalibrationsflüssigkeit, der erhobenen Kalibrationssignale (Schritt 5) und der erhobenen Meßgutsignale (Schritt 12). Anzeige der Meßgrößen.

## Patentansprüche

1. Verfahren zur Qualitätskontrolle eines Analysensystems bestehend aus einem portablen Analysator mit einsetzbaren Einmal-Kassetten, deren Probenraum optische und/oder elektrochemische Sensoren aufweist, wobei die Sensoren in Kontakt mit einem flüssigen Konditioniermedium gelagert werden, **dadurch gekennzeichnet**,
a) daß herstellungsbedingte Kenndaten der einzelnen Sensoren in den Analysator eingelesen werden bzw. gespeichert sind und die Kassette in den Analysator eingesetzt wird,
b) daß die sich im Probenraum befindliche Konditionierflüssigkeit durch ein gasförmiges Kalibriermedium ersetzt wird, wobei in Kontakt mit einzelnen der Sensoren verbleibende Reste der Konditionierflüssigkeit mit dem gasförmigen Kalibriermedium beaufschlagt werden,
c) daß die Kalibrationssignale der Sensoren simultan erfaßt werden,
d) daß das gasförmige Kalibriermedium durch eine Qualitätskontrollflüssigkeit ersetzt wird, welche im Hinblick auf jene chemischen und/oder physikalischen Parameter, welche die Kennlinie zumindest eines Sensors während der Kalibrierung oder Konditionierung beeinflussen, mit der Konditionierflüssigkeit - innerhalb der gewünschten Meßgenauigkeit für die zu messenden Probenbestandteile - übereinstimmt,
e) daß die Kontrollsignale der Sensoren simultan erfaßt werden,
f) daß aus den eingelesenen bzw. gespeicherten Kenndaten und den in den Punkten c) und e) erfaßten Kalibriations- und Kontrollsignalen Ist-Kontrollwerte für die einzelnen Sensoren ermittelt werden,
g) daß die Ist--Kontrollwerte mit den bekannten Soll-Kontrollwerten verglichen werden, und
h) daß anschließend an die Qualitätskontrolle mit derselben Kassette eine Probenmessung durchgeführt wird, falls die Ist-Kontrollwerte mit den Soll-Kontrollwerten innerhalb der gewünschten Meßgenauigkeit übereinstimmen.

2. Verfahren nach Anspruch 1, **dadurch gekennzeichnet,** daß die Sensoren in Punkt d) mit einer Qualitätskontrollflüssigkeit in Kontakt gebracht werden, welche im interessierenden Meßbereich und bei gegebener Meßtemperatur eine CO2/pH-Gleichgewichtskurve sowie einen pH20 aufweist, welche mit den entsprechenden Werten der Konditionierflüssigkeit - innerhalb der gewünschten Meßgenauigkeit für die zu messenden Probenbestandteile - übereinstimmen.

3. Verfahren nach Anspruch 2, **dadurch gekennzeichnet**, daß im interessierenden Meßbereich die Ionenstärke der Qualitätskontrollflüssigkeit bei einem vorgegebenen pH-Wert - innerhalb der gewünschten Meßgenauigkeit für die zu messenden Probenbestandteile - mit jener der Konditionierflüssigkeit übereinstimmt.

4. Verfahren nach Anspruch 2 oder 3, **dadurch gekennzeichnet**, daß der osmotische Druck der Qualitätskontrollflüssigkeit - innerhalb der gewünschten Meßgenauigkeit für die zu messenden Probenbestandteile - mit jener der Konditionierflüssigkeit übereinstimmt.

5. Verfahren zur Qualitätskontrolle eines Analysensystems bestehend aus einem portablen Analysator mit einsetzbaren Einmal-Kassetten, deren Probenraum optische oder elektrochemische Sensoren aufweist, wobei die Sensoren trocken gelagert werden, **dadurch gekennzeichnet**,
a) daß herstellungsbedingte Kenndaten der einzelnen Sensoren in den Analysator eingelesen werden bzw. gespeichert sind und die Kassette in den Analysator eingesetzt wird,
b) daß die Sensoren mit einem flüssigen Kalibriermedium beaufschlagt werden,
c) daß die Kalibrationssignale der Sensoren simultan erfaßt werden, wobei die Zeitspanne zwischen dem Erstkontakt der Sensoren mit der Kalibrierflüssigkeit und der Erfassung der Kalibrationssignale berücksichtigt wird,
d) daß die Sensoren mit einer Qualitätskontrollflüssigkeit kontaktiert werden, wobei ggf. zur Trennung der Kalibrierflüssigkeit von der Qualitätskontrollflüssigkeit ein Gas bekannter Zusammensetzung eingebracht wird, sowie daß die Qualitätskontrollflüssigkeit im Hinblick auf jene chemischen und/oder physikalischen Parameter, welche die Kennlinie zumindest eines Sensors während der Kalibrierung beeinflussen, mit der Kalibrierflüssigkeit - innerhalb der gewünschten Meßgenauigkeit für die zu messenden Probenbestandteile - übereinstimmt,
e) daß die Kontrollsignale der Sensoren erfaßt werden, wobei die Zeitpanne zwischen dem Erstkontakt der Sensoren mit der Kalibrierflüssigkeit und der Erfassung der Kontrollsignale berücksichtigt wird,
f) daß aus den eingelesenen bzw. gespeicherten Kenndaten und den in den Punkten c) und e) erfaßten Kalibrations- und Kontrollsignalen Ist-Kontrollwerte für die einzelnen Sensoren ermittelt werden,
g) daß die Ist-Kontrollwerte mit den bekannten Soll-Kontrollwerten verglichen werden, und
h) daß anschließend an die Qualitätskontrolle mit derselben Kassette eine Probenmessung durchgeführt wird, falls die Ist-Kontrollwerte mit den Soll-Kontrollwerten innerhalb der gewünschten Meßgenauigkeit übereinstimmen.

6. Verfahren nach Anspruch 5, **dadurch gekennzeichnet**, daß die Sensoren in Punkt d) mit einer Qualitätskontrollflüssigkeit in Kontakt gebracht werden, welche im interessierenden Meßbereich und bei gegebener Meßtemperatur eine CO2/pH-Gleichgewichtskurve sowie einen pH2O aufweist, welche mit den entsprechenden Werten der Kalibrierflüssigkeit in Punkt b) - innerhalb der gewünschten Meßgenauigkeit für die zu messenden Probenbestandteile - übereinstimmen.

7. Verfahren nach Anspruch 6, **dadurch gekennzeichnet**, daß im interessierenden Meßbereich die Ionenstärke der Qualitätskontrollflüssigkeit bei einem vorgegebenen pH-Wert - innerhalb der gewünschten Meßgenauigkeit für die zu messenden Probenbestandteile - mit jener der Kalibrierflüssigkeit übereinstimmt.

8. Verfahren nach Anspruch 6 oder 7, **dadurch gekennzeichnet**, daß der osmotische Druck der Qualitätskontrollflüssigkeit - innerhalb der gewünschten Meßgenauigkeit für die zu messenden Probenbestandteile - mit jener der Kalibrierflüssigkeit übereinstimmt.

## Claims

1. Method for quality control of an analyzing system consisting of a portable analyzer with insertable single-use cartridges whose sample chamber contains optical and/or electrochemical sensors, said sensors being stored in contact with a liquid conditioning medium, **characterized in that**
a) production-inherent characteristics of the individual sensors are read into the analyzer, or are already stored therein, and the cartridge is inserted into the analyzer;
b) the conditioning liquid contained in the sample chamber is replaced by a gaseous calibrating medium, residual amounts of the conditioning liquid remaining in contact with individual sensors being treated with the gaseous calibrating medium;
c) the calibration signals of the sensors are detected simultaneously;
d) the gaseous calibrating medium is replaced by a quality control liquid, which, as regards those chemical and/or physical parameters which influence the characteristic of at least one sensor during the calibrating or conditioning process, is identical with the conditioning liquid within the desired range of accuracy for the sample components to be measured;
e) the control signals of the sensors are detected simultaneosuly;
f) from the characteristics read into or known by the analyzer, and the calibration and control signals detected in items c) and e), instantaneous control values are determined for the individual sensors;
g) the instantaneous control values are compared with the known target control values;
h) subsequent to quality control the same cartridge is employed for sample measurement, if the instantaneous control values are identical with the target control values within the desired range of accuracy.

2. Method according to Claim 1, **characterized in that** the sensors in item d) are brought into contact with a quality control liquid whose CO₂/pH equilibrium curve and pH₂O in the interesting measuring region and at a given measuring temperature are identical with the respective values of the conditioning liquid within the desired range of accuracy for the sample components to be measured.

3. Method according to Claim 2, **characterized in that** in the interesting measuring region and at a given pH the ionic strength of the quality control liquid is identical with that of the conditioning liquid, within the desired range of accuracy for the sample components to be measured.

4. Method according to Claim 2 or 3, **characterized in that** the osmotic pressure of the quality control liquid is identical with that of the conditioning liquid, within the desired range of accuracy for the sample components to be measured.

5. Method for quality control of an analyzing system consisting of a portable analyzer with insertable single-use cartridges whose sample chamber contains optical and/or electrochemical sensors, said sensors being dry-stored, **characterized in that**
a) production-inherent characteristics of the individual sensors are read into the analyzer, or are already stored therein, and the cartridge is inserted into the analyzer;
b) the sensors are subjected to a liquid calibrating medium;
c) the calibration signals of the sensors are detected simultaneously, taking into account the time lapse between the primary contact of sensors and calibrating liquid and the detection of the calibration signals ;
d) the sensors are contacted with a quality control liquid, with the possible addition of a gas of known composition for the purpose of separating the calibrating liquid from the quality control liquid, which latter, as regards those chemical and/or physical parameters that influence the characteristic of at least one sensor during calibration, is identical with the calibrating liquid within the desired range of accuracy for the sample components to be measured;
e) the control signals of the sensors are detected, taking into account the time lapse between the primary contact of sensors and calibrating liquid and the detection of the calibration signals;
f) from the characteristics read into or known by the analyzer, and the calibration and control signals detected in items c) and e), instantaneous control values are determined for the individual sensors;
g) the instantaneous control values are compared with the known target control values;
h) subsequent to quality control the same cartridge is employed for sample measurement, if the instantaneous control values are identical with the target control values within the desired range of accuracy.

6. Method according to Claim 5, **characterized in that** the sensors in item d) are brought into contact with a quality control liquid whose CO₂/pH equilibrium curve and pH₂O in the interesting measuring region and at a given measuring temperature are identical with the respective values of the calibrating liquid within the desired range of accuracy for the sample components to be measured.

7. Method according to Claim 6, **characterized in that** in the interesting measuring region and at a given pH the ionic strength of the quality control liquid is identical with that of the calibrating liquid, within the desired range of accuracy for the sample components to be measured.

8. Method according to Claim 6 or 7, **characterized in that** the osmotic pressure of the quality control liquid is identical with that of the calibrating liquid, within the desired range of accuracy for the sample components to be measured.

## Revendications

1. Procédé de contrôle de qualité d'un système d'analyse formé d'un analyseur portable avec une cassette à usage unique dont l'enceinte à échantillons comporte des capteurs optiques et/ou électrochimiques, les capteurs étant stockés en contact avec un milieu de conditionnement liquide,
caractérisé en ce que
a) les caractéristiques de fabrication des différents capteurs sont enregistrées dans l'analyseur ou sont mémorisées dans celui-ci et la cassette est placée dans l'analyseur,
b) le liquide de conditionnement qui se trouve dans l'enceinte à échantillons est remplacé par un milieu de calibrage gazeux et on fait agir le milieu de calibrage gazeux sur les résidus du liquide de conditionnement qui reste en contact avec les différents capteurs,
c) on saisit simultanément les signaux de calibrage des capteurs,
d) on remplace le milieu de calibrage gazeux par un liquide de contrôle de qualité qui coïncide du point de vue des paramètres chimiques et/ou physiques respectifs influençant la courbe caractéristique d'au moins un capteur pendant le calibrage ou le conditionnement, avec le liquide de conditionnement (à l'intérieur de la précision de mesure souhaitée pour les composants à mesurer de l'échantillon),
e) on saisit simultanément les signaux de contrôle des capteurs,
f) à partir des caractéristiques enregistrées ou mémorisées et des signaux de calibrage et de contrôle saisis sous les points c) et e), on détermine des valeurs de contrôle réelles pour les différents capteurs,
g) on compare les valeurs de contrôle réelles aux valeurs de contrôle de consigne, connues et,
h) on effectue une mesure échantillon avec la même cassette à la suite d'un contrôle de qualité si les valeurs de contrôle réelles coïncident avec les valeurs de contrôle de consigne à l'intérieur de la plage de précision de mesure souhaitée.

2. Procédé de contrôle de qualité selon la revendication 1,
caractérisé en ce qu'
on met les capteurs du point d) en contact avec un liquide de contrôle de qualité qui présente dans la plage de mesure concernée et pour une température de mesure donnée, une courbe d'équilibre CO2/pH ainsi qu'un pH2O correspondant aux valeurs respectives du liquide de conditionnement à l'intérieur de la pression de mesure souhaitée pour des composants d'échantillons à mesurer.

3. Procédé de contrôle de qualité selon la revendication 2,
caractérisé en en ce que
la plage de mesure concernée, l'intensité ionique du liquide de contrôle de qualité pour une valeur pH prédéterminée coïncident avec des intensités du liquide de conditionnement respectif à l'intérieur de la précision de mesure souhaitée pour les composants d'échantillons à mesurer.

4. Procédé de contrôle de qualité selon les revendications 2 ou 3,
caractérisé en ce que
la pression osmotique du liquide de contrôle de qualité correspond à celle du liquide de conditionnement à l'intérieur de la précision de mesure souhaitée pour les composants d'échantillons à mesurer.

5. Procédé de contrôle de qualité d'un système d'analyse formé d'un analyseur portable dans lequel est placée une cassette à usage unique, et dont l'espace à échantillons comporte des capteurs optiques et électrochimiques, ces capteurs étant stockés à sec,
caractérisé en ce que
a) les données caractéristiques liées à la fabrication des différents capteurs sont enregistrées ou mémorisées dans l'analyseur et on place la cassette dans l'analyseur,
b) on applique un milieu de calibrage liquide aux capteurs,
c) on saisit simultanément les signaux de calibrage des capteurs et l'intervalle entre le premier contact des capteurs avec le liquide de calibrage et la saisie des signaux de calibrage est pris en compte,
d) on met les capteurs en contact avec un liquide de contrôle de qualité et le cas échéant pour séparer le liquide de calibrage du liquide de contrôle de qualité, on introduit un gaz de composition connu et on influence le liquide de contrôle de qualité du point de vue du paramètre chimique et/ou physique respectif influençant la courbe caractéristique d'au moins un capteur pendant le calibrage, qui correspond au liquide de calibrage à l'intérieur de la précision souhaitée des composants d'échantillons à mesurer,
e) on saisit les signaux de contrôle des capteurs et on tient compte de l'intervalle de temps entre le premier contact des capteurs avec le liquide de calibrage et la saisie des signaux de contrôle,
f) à partir des données enregistrées ou mémorisées et des signaux de calibrage et de contrôle saisis sous les points c) et e), on détermine des valeurs de contrôle réelles pour les différents capteurs,
g) on compare les valeurs de contrôle réelles aux valeurs de contrôle de consigne, connues et,
h) après les contrôles de qualité, on fait avec la même cassette une mesure échantillon au cas où les valeurs de contrôle réelles coïncideraient avec les valeurs de contrôle de consigne à l'intérieur de la précision de mesure souhaitée.

6. Procédé de contrôle de qualité selon la revendication 5,
caractérisé en ce que
les capteurs du point d) sont mis en contact avec un liquide de contrôle de qualité qui présente dans la plage de mesure concernée et pour la température de mesure donnée, une courbe d'équilibre CO2/pH ainsi qu'un pH2O qui correspondent aux valeurs respectives du liquide de calibrage sous le point b) à l'intérieur de la précision de mesure souhaitée pour les composants d'échantillons à mesurer.

7. Procédé de contrôle de qualité selon la revendication 6,
caractérisé en ce que
dans la plage de mesure concernée, l'intensité ionique du liquide de contrôle de qualité correspond à celle du liquide de calibrage pour une valeur pH prédéterminée à l'intérieur de la précision de mesure souhaitée pour les composants d'échantillons à mesurer.

8. Procédé de contrôle de qualité selon les revendications 6 ou 7,
caractérisé en ce que
la pression osmotique du liquide de contrôle de qualité correspond à celle du liquide de calibrage à l'intérieur de la précision de mesure souhaitée pour les composants d'échantillons à mesurer.
